Europäisches Patentamt

(19) European Patent Office

Office européen des brevets

(11) Publication number: **0 034 442**
A2

(12) # EUROPEAN PATENT APPLICATION

(21) Application number: **81300472.8**

(22) Date of filing: **04.02.81**

(51) Int. Cl.³: **C 07 C 45/34**
C 07 C 47/22, C 07 C 51/25
C 07 C 57/055
//B01J23/76

(30) Priority: **08.02.80 US 119608**

(43) Date of publication of application:
**26.08.81 Bulletin 81/34**

(84) Designated Contracting States:
**BE CH DE FR GB IT LI LU NL SE**

(71) Applicant: **THE STANDARD OIL COMPANY**
**Midland Building**
**Cleveland, Ohio 44115(US)**

(72) Inventor: **Bigler, Kenneth LeRoy**
**273 Chatham Drive**
**Aurora Ohio 44202(US)**

(72) Inventor: **Miller, Arthur Francis**
**4827 South Sedgewick Road**
**Lyndhurst Ohio 44124(US)**

(74) Representative: **Smith, Sydney et al,**
**Elkington and Fife High Holborn House 52/54 High**
**Holborn**
**London WC1V 6SH(GB)**

(54) Process for the preparation of unsaturated aldehydes and unsaturated acids.

(57) A process for the preparation of unsaturated aldehydes and unsaturated acids by contracting the respective feed with an oxidation catalyst through the reaction zone of a transport line reactor while maintaining the linear gas velocity between 5-25 ft./sec. (1,50-7.50 metres/sec.) to achieve a substantially plug flow of through the reactor. Propylene and isobutylene are oxidised to acrolein and methacrolein which are oxidised to acrylic and methacrylic acid.

EP 0 034 442 A2

TITLE MODIFIED
see front page

- 1 -

TITLE

"PROCESS FOR PREPARING ALDEHYDES
AND ACIDS"
-----------------------------------

This invention relates to a process for preparing aldehydes and acids, more particularly unsaturated aldehydes and acids such as acrolein and acrylic acid.

Unsaturated aliphatic acids such as acrylic acid or methacrylic acid are prepared, typically, in a two-stage process. First, propylene and air are passed over an oxidation catalyst to form acrolein with minor amounts of acrylic acid. The reactor effluent from this first stage is then passed over a second oxidation catalyst to promote the reaction of acrolein to acrylic acid.

Reactor systems for vapour phase processes can be in fixed or fluid-bed form. The choice depends on the process itself, the type of catalyst used, etc. Each of these forms of reactors has advantages and disadvantages.

Fixed-bed reactors tend to provide excellent gas solids contacting, less catalyst than for a fluid bed, and a smaller reactor. Fixed-bed reactors also allow the grading of catalysts within the reactor, and different types of catalyst may be installed at different locations. However, in reactions in which there is a large amount of heat released or absorbed, fixed-bed reactors require a large amount of heat transfer service to adequately control the reaction temperature, and cannot be controlled isothermally. The fixed-bed particles are normally much larger than fluid-bed particles, and therefore are more sensitive to reactant diffusion complication. Finally, fixed-bed reactors

- 2 -

generally require efficient mixing of all reactants prior to entering the catalyst bed.

Fluid-bed reactors result where gas is passed through the reactor at a sufficient velocity to "fluidize" catalyst particles. Because of this fluidized state, fluid beds have excellent heat conductivity and require only moderate amounts of heat transfer area. The feeds do not have to be well mixed for this may take place within the bed. However, along with increased reactor size, a fluid bed could tend to bypass reactant gas due to bubble formation.

A third type of reactor system is known as "fast fluidization" or "transport line reactor". This type of reactor is characterized in that the reactant gases and catalyst move through a tubular reactor in substantially plug flow while within this plug flow there is extreme turbulence and substantial back mixing of catalyst. Such reactor systems have found use in petroleum refining such as catalytic cracking. U.S. Patent No. 4,102,914 discloses a fast fluidization system for the preparation of acrylonitrile.

It has been found that transport line reactors can be applied to the process for the preparation of unsaturated acids with excellent results.

According to the present invention a transport line reactor system is applied to either or both of the reactions for the preparation of aldehydes and the preparation of carboxylic acids.

The invention therefore provides in one aspect a process for preparing unsaturated aldehydes from unsaturated olefins comprising passing said olefin and molecular oxygen in the vapour phase into a transport line reactor with a solid oxidation catalyst while maintaining the linear gas velocity between 5-25 ft./ sec. (1.50-7.50 metres/sec.) to achieve a substantially plug flow of catalyst and olefin within the reactor.

The invention provides in another aspect a process for preparing unsaturated carboxylic acids from unsaturated aldehydes comprising passing said unsaturated aldehydes in the vapour phase with a solid oxidation catalyst into a transport line reactor, while maintaining the linear gas velocity between 5-25 ft./sec. (1.50-7.50 metres/sec.) to achieve a substantially plug flow of catalyst and unsaturated aldehyde.

The invention provides in yet another aspect a process for preparing unsaturated carboxylic acid in two stages, the first stage converting an unsaturated olefin to an unsaturated aldehyde, and the second stage converting the unsaturated aldehyde to the unsaturated carboxylic acid, wherein both stages utilize a transport line reactor having a linear gas velocity between 5-25 ft/sec. (1.50-7.50 metres/sec.) within the reactor to achieve a substantially plug flow through the reactor.

While the present invention is applicable to the vapour phase production of any unsaturated aldehyde and/or carboxylic acid, it is preferred that the unsaturated olefins utilized in the initial step of the reaction be propylene or isobutylene. This results in the formation of preferred unsaturated aldehydes of acrolein and methacrolein, and preferred unsaturated carboxylic acids of acrylic acid and methacrylic acid.

The oxidation catalysts useful in this process are well known in the art. For example, U.S. Patent No. 3,859,358 discloses catalyst comprising the oxide complexes of uranium and molybdenum; U.S. Patent No. 3,171,859 describes catalyst of the oxides of iron,

- 4 -

bismuth, phosphorus and molybdenum; U.S. Patent No. 3,642,930 discloses catalyst comprising an alkali metal, bismuth, iron and molybdenum, U.S. Patent No. 4,138,366 discloses antimony, molybdenum and vanadium catalyst. While the catalyst of each stage can be similar, typically first-stage catalyst contain at least bismuth and molybdenum oxides whereas second-stage catalyst contain vanadium and molybdenum oxides.

The manner of making these catalysts are well known in the art and will not be described further here. There are, however, properties of the catalyst that are preferred for use in transport line reactors. In particular the catalyst should be fairly attrition resistant because the catalyst receives far more handling during the fast fluidization than found in other types of reactor systems. Finally, it is preferred that the catalyst have high activity. While this is not crucial to the catalyst use in the transport line reactor, the short contact time found in such systems, make this property desirable.

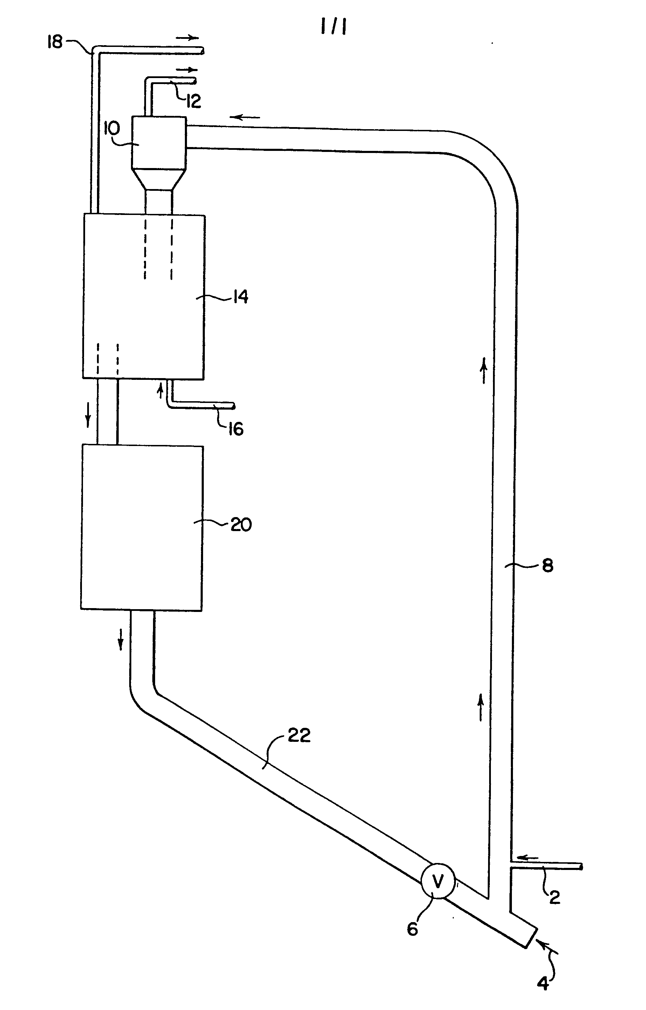

The invention will now be further described with reference to the single figure of the accompanying drawing, which is a schematic representation of a transport line reactor in use according to the present invention.

Referring to the drawing reactor 8 consists of an elongated tube that is typically vertical but can be in a horizontal position. Catalyst enters reactor 8 through slide valve 6 and is mixed with air through line 4 and propylene through line 2. The air and the propylene are at a sufficient flow rate to achieve a linear gas velocity through the reactor of between 5-25 ft./sec. (15.0-7.50 metres/sec.). At this velocity, the catalyst and reactant gases move through reactor 8 in a substantially plug flow manner. Within this section of plug flow that is moving through the reactor, however, the catalyst particles are characterized by extreme turbulence and substantial back mixing.

This plug flow of catalyst and reactant gases move through the reactor and after reacting, are passed to cyclone 10. The purpose of cyclone 10 is to separate the solid catalyst from the gaseous reactor effluent. The reactor effluent leaves the cyclone in line 12 while the catalyst drops down from the cyclone into stripper 14.

The purpose of stripper 14 is twofold. It has been discovered that the catalyst builds up carbon as it passes through the transfer line reactor, and also absorbs a substantial amount of the desired product of the reaction. These products may be recovered by passing a stripping gas such as steam through line 16 into the stripper. The products of the reaction are then stripped off the catalyst and leave the stripper through line 18 which can be combined with the majority of the reactor effluent leaving in line 12. Fluidizing gas such as nitrogen may also be injected into the stripper to maintain the catalyst in the fluidized state.

After stripping, the catalyst then passes to hopper 20. Hopper 20 serves as a reservoir for the catalyst as it moves through the system. Fluidizing gas should also be injected into the hopper to maintain the catalyst in a fluidized state.

The catalyst then passes from hopper 20 through line 22 and slide valve 6 back to the reactor. Thus the catalyst is constantly recycled throughout the process with minimum losses.

For the first stage reaction of unsaturated olefins to unsaturated aldehydes, the temperature of

- 6 -

the reactor should be maintained between 500°-900°F (260-482°C) with the preferred temperature range of 600-800°F (316-426°C). The propylene to air ratio useful in this system is 1:4 to about 1:16, with a ratio of about 1:10 being preferred. The reactor pressure is typically O to no more than 100 psig (1-8 kg/cm$^2$) with about 0-50 psig (1-5 kg/cm$^2$) being preferred.

For the second stage unsaturated aldehyde to carboxylic acid reaction, temperatures in the range of 300 to 750°F (149-399°C) are maintained and 390-660°F (199-349°C) preferred. The molar ratio of oxygen to acrolein can be between 0.2 to about 2.0 with 0.5 to 1.5 being preferred.

The apparent contact time employed in the process is not especially critical. Contact times in the range of 0.1 to 50 seconds may be employed. The apparent contact time may be defined as the length of time in seconds which a unit volume of gas measured under the conditions of reaction is in contact with the apparent unit volume of catalyst. It may be calculated, for instance, from the apparent volume of the catalyst bed, the average temperature and pressure of the reaction and the flow rates in the vessel of the components of the reaction mixture. The optimum contact time will, of course, vary depending upon the olefin being treated and the catalyst being used but, in general, it may be said that a contact time of 1 to 10 seconds is preferred.

The catalyst density should be between 1-25 lbs/ft$^3$ (16-400 kg/m$^3$) with a preferred density of 5-20 lbs/ft$^3$ (80-320 kg/m$^3$). The catalyst density is defined as the total weight of catalyst in the reaction zone divided by the volume of the reaction zone and is calculated from the reactor differential pressure.

Examples 1-4

Preparation of Acrolein

A Transport Line Reactor as shown in the drawing was utilized for the preparation of acrolein. The reactor was 42 ft. 12.80 metres long with a one inch diameter. Nitrogen was used as a fluidizing gas in both the stripper and the hopper.

An oxidizing catalyst having the components of 50% ($K_{0.07}Co_{4.5}Fe_3Ni_{2.5}BiP_{0.5}Mo_{12}O_x$) 50% $SiO_2$ was prepared with prior art methods. Approximately 60 lbs. (27 kgs) of this catalyst was charged to the Transport Line Reactor For Examples 1-4, the catalyst flow rate was 20 lbs/minute (0.148 kg/sec.) with a reactor gas velocity of 8 ft./sec. (2.40 m/sec.) giving a reactor catalyst density of 11.4 lbs/ft 15.4 kg/m. Propylene was fed to the unit at a rate of 2.86 standard litres per minute (SLM) which was approximately .75 lbs/hr (0.337 kg/hr). The results of different temperatures and air and nitrogen rates are shown in Table 1.

TABLE  I

Preparation of Acrolein

| Example | Temp °C | Feed $N_2/AIR/C_3^=$ | Per Pass Conv. % Acrolein | Acrylic |
|---------|---------|----------------------|---------------------------|---------|
| 1 | 360 | 16.8/10.7/1 | 62.0 | .97 |
| 2 | 380 | 20.3/9.8/1 | 54.2 | 3.2 |
| 3 | 380 | 15.4/9.8/1 | 67.5 | 5.8 |
| 4 | 380 | 24.5/10.7/1 | 71.6 | 3.6 |

| Example | Selectivity % Acrolein | Acrylic | Total Conversion % |
|---------|------------------------|---------|--------------------|
| 1 | 80.9 | 1.27 | 76.6 |
| 2 | 76.9 | 4.5 | 71.2 |
| 3 | 80.9 | 6.95 | 83.4 |
| 4 | 86.3 | 4.3 | 82.9 |

- 9 -
### Examples 5-8
### Effect of Stripping Catalyst

Examples 5-8 show the effect of utilizing stripping steam in the stripper to remove absorbed products and carbon built up on the catalyst.

Examples 5 and 6, utilizing the apparatus of Example 1, show that when stripping steam is used in the stripper both the total conversion and the per pass conversion to products increase when acrolein is being prepared from propylene.

Examples 7-8 show that stripping steam has the same effect when a Transport Line Reactor is utilized for the preparation of acrylic acid from acrolein.

### Table II
### Effect of Stripping

| Example | Reaction | Stripping |
|---------|----------|-----------|
| 5 | Acrolein | no |
| 6 | " | yes |
| 7 | Acrylic Acid | no |
| 8 | " " | yes |

| Example | Total Conver., % | Product Per Pass, % | Sel., % |
|---------|------------------|---------------------|---------|
| 5 | 73.1 | 59.7 | 82.6 |
| 6 | 82.8 | 73.0 | 88.1 |
| 7 | 83.9 | 64.7 | 77.1 |
| 8 | 90.7 | 73.0 | 80.5 |

### Examples 9-10
### Preparation of Acrylic Acid

The reactor effluent from a first stage propylene to acrolein process, having the composition shown in Table III, was fed to a Transport Line Reactor for the preparation of acrylic acid. A catalyst having the

- 10 -

composition 40% $[Mo_{12}V_3W_{1.2}Cu_2Sn_{0.5}Ox]$ 60% $SiO_2$ was charged to the reactor in a manner similar to that of Example 1. The first stage effluent was fed to the TLR at a rate of 53.7 SLM for Example 9 and 52.2 SLM for Example 10. The reactor was operated at a temperature of approximately 500°F (260°C) 9.0 SLM of nitrogen and 10.0 SLM of steam was fed to the stripper to both fluidize and strip the products of reaction from the catalyst. Additionally, 6.8 SLM of air was fed to the hopper to fluidize the catalyst. Finally, 8.5 SLM of air and 11.4 SLM of nitrogen was fed to the reactor along with the catalyst and the first stage effluent being used as feed.

The results of these examples are shown in Table IV. The results presented in the Table are the sum of the products leaving from the cyclone and the stripper.

### TABLE III

#### Feed to Acrylic Acid TLR

| Component | Mol % |
|---|---|
| Acrylic Acid | 2.68 |
| Acrolein | 5.92 |
| Co + $CO_2$ | 3.81 |
| Acetic Acid | 0.23 |
| $N_2$ | 67.28 |
| $H_2O$ | 17.50 |
| Propane | 0.54 |
| Propylene | 0.66 |
| Oxygen | 1.38 |

- 11 -

Table IV

Preparation of Acrylic Acid

| Example | Per Pass Conversion, % | | |
| | Acrylic | Acrolein | (unreacted) |
| --- | --- | --- | --- |
| 9 | 65.0 | 4.8 | |
| 10 | 59.6 | 1.7 | |

- 12 -

CLAIMS

1.  A process for preparing unsaturated aldehydes from unsaturated olefins which comprises
passing said olefin and molecular oxygen, in the
vapour phase, into a transport line reactor with a
solid oxidation catalyst, while maintaining the linear
gas velocity between 5-25 ft./sec., 1.50-7.50 metres/
sec. to achieve a substantially plug flow of catalyst
and olefin within the reactor.

2.  A process as claimed in claim 1 characterized in that the unsaturated olefin is selected from
propylene and isobutylene.

3.  A process as claimed in claim 1 or claim 2
characterized in that the oxidation catalyst is an
oxide or oxide complex containing at least molybdenum
oxide.

4.  A process as claimed in claim 3 characterized in that the oxidation catalyst contains at least
bismuth and molybdenum oxides.

5.  A process for preparing unsaturated
carboxylic acids from unsaturated aldehydes which
comprises passing said unsaturated aldehydes in the
vapour phase with a solid oxidation catalyst into a
transport line reactor, while maintaining the linear
gas velocity between 5-25 ft./sec. 1.50-7.50 metres/sec
to achieve a substantially plug flow of catalyst and
unsaturated aldehydes.

6.  A process as claimed in claim 5 characterized in that the unsaturated aldehyde is selected from
acrolein and methacrolein.

7.  A process as claimed in claim 5 or claim 6
characterized in that the solid oxidation catalyst is
an oxide or oxide complex of at least molybdenum oxide.

8. A process as claimed in claim 7 characterized in that the solid oxidation catalyst comprises at least vanadium and molybdenum oxide.

9. A process as claimed in any of claims 1 to 8 characterized in that the solid oxidation catalyst density is between 1-25 lbs./ft$^3$ (16-400 kg/m$^3$).

10. A process as claimed in any of claims 1 to 9 characterized in that the transport line reactor comprises a reaction zone, followed by means for separating the solid oxidation catalyst from the resultant reaction gases and catalyst exiting said reaction zone.

11. A process as claimed in claim 10 characterized in that after separating the solid oxidation catalyst, the transport line reactor has means for stripping the catalyst to remove aldehydes.

12. A process as claimed in claim 10 or claim 11 characterized in that the catalyst is recycled back to the reaction zone.

13. A process for preparing unsaturated carboxylic acids in two stages, the first stage converting an unsaturated olefin to an unsaturated aldehydes, and the second stage converting the unsaturated aldehydes to the unsaturated carboxylic acid, wherein both stages utilize a transport line reactor having a linear gas velocity between 5-25 ft./sec. (1.50-7.50 metres/sec.) within the reactor to achieve a substantially plug flow through the reactor.

14. A process as claimed in claim 13 characterized in that the unsaturated olefin is selected from propylene and isobutylene, the unsaturated aldehyde is selected from acrolein and methacrolein, and the unsaturated carboxylic acid is selected from acrylic acid and methacrylic acid.